# EUROPEAN PATENT APPLICATION

(11) **EP 4 420 675 A1**
(43) Date of publication of application: **28.08.2024**
(21) Application number: 22860493.0
(22) Date of filing: 23.08.2022
(51) Int. Cl.: A61K 39/00, C07K 1/107, A61K 39/395

(54) **EPITOPE MODIFICATION**

(30) Priority: 23.08.2021 CN 202110968141
(71) Applicant: Nantong Yichen Biopharma. Co. Ltd., Nantong, Jiangsu 226000 (CN)
(72) Inventor: WANG, Feng, Nantong, Jiangsu 226000 (CN); ZHU, Chaoyang, Nantong, Jiangsu 226000 (CN); XU, Liang, Nantong, Jiangsu 226000 (CN)
(74) Representative: HGF
(86) International application number: PCT/CN2022/114250
(87) International publication number: WO 2023/025147

(57) **Abstract**

The present invention relates to epitope modification based on a chemical cross-linking reactive group and the use thereof in changing the immunogenicity of an antigen and increasing animal immune response to a target epitope of an antigen. The present invention relates to the administration of a mutant antigen incorporated by a group with chemical cross-slinking activity on the target epitope of a wild-type antigen or derivative thereof to an animal, wherein the antibody reaction in the animal is directed and enriched to the target epitope of the mutant antigen. Provided are a method for selecting antibodies against a target epitope of an antigen and the antibodies obtained thereby; further provided is the use of the method in the preparation of a vaccine for preventing and treating diseases.

## Description

### Technical field

The invention belongs to the technical field of biophamaceuticals.

### Background

Antibodies are important components of vertebrate adaptive immunity and play crucial roles in preventing bacterial and viral infections by neutralizing most foreign harmful substances (Tomita and Tsumoto, 2010). The advantages of monoclonal antibodies, such as high affinity, high specificity, good bio-compatibility, and cellular effects, make them an important therapy for a variety of diseases, such as autoimmune diseases and cancer (Sharma and Allison, 2015; Singh et al., 2018; Sliwkowski and Mellman, 2013). Mouse hybridoma technology is one of the most common methods for producing high-affinity monoclonal antibodies (Schwaber, 1982; Zhang, 2012). In recent years, with the application of transgenic mice with inserted human antibody gene fragments in production of human antibodies, mouse immunization and screening of downstream monoclonal antibodies have become the main methods for identifying and developing therapeutic antibodies (Brüggemann, 2001; Brüggemann et al., 2015; Taylor et al., 1992). However, this approach is not always effective in generating functional antibodies (Kellermann and Green, 2002). A therapeutic antibody must bind a specific epitope of the target antigen to perform its intended function, such as blocking ligand-receptor interactions (i.e. as an antagonist), or inducing receptor-mediated downstream signaling (i.e. as an agonist) (Weiner et al., 2010). Unfortunately, therapeutically useful epitopes may only occupy a small fraction of the entire surface of antigens (Sercarz et al., 1993). Through current methods, the whole antigen as immunogen would only eliciting little antibodies against the desired epitope. Moreover, non-functional but immunodominant B-cell epitopes on the target antigen further reduce this possibility. It requires high-throughput screening to select functional antibodies from numerous monoclonal antibodies (Pasqualini and Arap, 2004). To make things worse, it may be difficult to elicit antibody responses against functional epitopes of human antigen that share high sequence homology and structural similarity to its mouse counterpart due to host immune tolerance (Goodnow et al., 1988; Hartley et al., 1993; Nemazee, 2017), which is very common in many important drug targets such as GPCRs. The application of epitope sequence as immunogen would only elicit antibodies against the linear epitope, which would not bind to conformational epitope in the target (Xu et al., 2018)

To overcome this challenge, scientists have tried several approaches to increase the odds of screening for functional antibodies. Viktoriya Dubrovskaya et al obtained broadly neutralizing antibodies by using the modified glycosylated epitopes of the HIV envelope protein as immunogen (Dubrovskaya et al., 2019). Fabian Sesterhenn et al. fused respiratory syncytial virus (RSV) epitopes into antigens, and obtained neutralizing antibodies that specifically bind RSV epitopes (Sesterhenn et al, 2020). However, these methods are only feasible in specific cases, and it is difficult to apply them to the screening of antibodies against specific epitopes of other antigens.

The engineered tRNA/aaRS orthogonal pair obtained through screening and selection can specifically recognize unnatural amino acids (UAA) and incorporate them into the coding site of the target in living host cells (Wang et al., 2001). Various chemical groups on UAA side chains can endow proteins with new functions, so they have shown powerful applications in the study of protein structure and function, cell imaging, therapeutic protein conjugation, and many other fields (Chin, 2014 Year). In our previous work, we used p-benzoyl-1-phenylalanine (pBpa)-incorporated antigens to successfully select antibodies that bind to the desired epitope of the target antigen from the antibody phage display library by photocrosslinking panning(Chen et al., 2020), suggesting that this may be the first antibody screening method available for desired epitopes of all types of antigens. However, this method relies on panning of phage libraries and affinity maturation of the screened antibodies in vitro.

In summary, none of the current methods can selectively elicit antibody responses to antigen-specific conformational epitopes in vivo.

### Summary

In view of the fact that antibodies produced by whole antigens-immunized mice are usually enriched to immuno-dominant B cell epitopes, which usually reduces the possibility of obtaining antibodies against specific epitopes which often induced low immune responses, the present invention has developed a new method of enriching antibodies against specific epitopes by modifying the specific epitopes with chemical cross-linking reactive groups as to increase their immunogenicity, thereby changing the antibody spectrum elicited by the antigen after immunization. The present invention further provides a method for enhancing the immune response of the animal against the specific epitope of the target antigen and enriching the antibodies against the specific epitope of the target antigen.

Antibodies that bind to specific epitopes of target antigens can be elicited and identified by the present invention easily and quickly, that is, utilizing incorporation of chemical cross-linking reactive groups(such as Nε-acryloyl or Nε-crotonyl) into specific epitope (such as E64) of the target antigen (such as IL-1β) to create "super" immunodominant B cell epitopes by ways (such as introducing natural or non-canonical amino acids containing chemical cross-linking active groups into the amino acid sequence of specific epitopes, or introducing chemical cross-linking reactive groups into specific epitopes of target antigen) as immunogen to direct and enrich antibody responses against specific epitopes of the target antigen in mice. In specific examples, this effect is so prominent that almost all clones randomly selected from IL-1βE64AK and IL-1βE64CK-immunized phage libraries were capable of binding to the specific epitope. In contrast, clones from WT IL-1β(the wild-type antigen) or IL-1β mutant (is incorporated by a non-canonical amino acid without cross-linking activity)-immunized mice rarely bind to the specific epitope. In addition, the difference in cross-linking activity between IL-1βE64AK and IL-1βE64CK lead to the difference of clone sequences from immunization, suggesting that it is feasible to tune antibody affinity and sequence diversity by adjusting the reactivity and structure of chemical cross-linking reactive groups.

The present invention further found that the directed antibody responses induced by AK or CK-incorporated epitopes is not limited by sequence: IL-1β antigen with CK incorporation into other sites as immunogen can effectively induce high-titer antibodies against specific epitopes. When the specific epitope is located at the binding interface of IL-1β and IL1RI, specific antibodies elicited by AK-incorporated epitopes can effectively block IL-1β from activating its receptor. Basing on this observation, epitope-directed antibody responses can be applied to vaccine development by enhancing effective immune responses to functional epitopes.

The present invention further found that the mutant polypeptide (PTN-CK), obtained by introducing CK with a chemical cross-linking active group (such as Nε-crotonyl) into a specific position of the polypeptide (such as PTN, a polypeptide of the Phaeodactylum tricomutum protein) ), can significantly enhance the immunogenicity of PTN itself (PTN-WT), and the antibody titer against PTN-CK in the serum of PTN-CK-immunized mice was significantly higher than that of PTN-WT-immunized mice. Using PTN-CK-coupled or PTN-WT-coupled carrier protein (KLH) as immunogen, the antibody titer against KLH in the serum of KLH-PTN-CK immunized mice was significantly reduced, while antibodies directed against PTN- CK (compared to KLH-PTN-WT) were enriched.

One aspect of the present invention provides an agent for immunizing animals, wherein said agent comprising:
- an immunologically effective amount of a mutant antigen, wherein the mutant antigen is incorporated by group with chemical cross-linking activity or a derivative thereof on one or more target epitopes in the wild-type antigen, and
- a physiologically acceptable carrier,
wherein the agent elicits or enhances the production of antibodies against the one or more target epitopes in the animal after being administered the mutant antigen.

In some embodiments, the group with chemical cross-linking activity is Nε-crotonyl or Nε-acryloyl.

In some embodiments, the group with chemical cross-linking activity is a natural amino acid or a non-canonical amino acid.

In some embodiments, the non-canonical amino acid with a chemically cross-linking active group is Nε-crotonyl-L-lysine (CK) or Nε-acryloyl-L-lysine (AK).

In some embodiments, the group with chemical cross-linking activity or derivatives thereof is incorporated by genetic codon expansion or by chemical synthesis.

In some embodiments, the animal is a rodent, a non-human mammal, or a mammal.

In some embodiments, the wild-type antigen is a soluble protein, a soluble polypeptide, a transmembrane protein expressed on a phospholipid membrane, or a polypeptide expressed on a phospholipid membrane.

In some embodiments, the agent is a agent for antibody preparation.

In some embodiments, the agent is a prophylactic or therapeutic agent.

In some embodiments, the agent is a vaccine composition.

In some embodiments, the agent comprises an immunologically effective amount of IL-1β mutant antigen; in some other embodiments, the E64 position of the IL-1β mutant antigen is incorporated with AK; in some other embodiments, The E64 position of the IL-1β mutant antigen is incorporated with CK.

In some embodiments, the agent comprises an immunologically effective amount of the novel corona virus S protein RBD mutant antigen; in some embodiments, K417 epitope, L452 epitope, L452 epitope, Y453 epitope, E484 epitope or N501 epitope of the novel corona virus S protein RBD mutant antigen is incorporated with CK.

In some embodiments, the agent comprises an immunologically effective amount of PTN-CK; in some embodiments, the agent comprises an effective amount of PTN-CK which is coupled to a carrier protein; in some embodiments, the carrier protein is hemocyanin KLH; in some embodiments, the agent comprises an effective amount of KLH-PTN-CK.

Another aspect of the present invention provides an agent for enhancing the immune response in animals, said agent comprising:
- an immunologically effective amount of a mutant antigen, which is formed by incorporation a chemical cross-linking active group or a derivative thereof on one or more target epitopes in the wild-type antigen, and
- a physiologically acceptable carrier.

Another aspect of the present invention provides a method for modulating the immunogenicity of an antigen, the method comprising incorporating a group with chemical cross-linking activity or a derivative thereof on one or more target epitopes of the antigen.

In some embodiments, the modulation results in changes in the profile of antigen-elicited antibodies.

Another aspect of the present invention provides a method for enhancing an animal's immune response against target epitope of an antigen, wherein the method comprising: administering a certain amount of mutant antigen to the animal, wherein the mutant antigen is incorporated with chemically cross-linked active groups or derivatives on the target epitope of the wild-type antigen.

Another aspect of the present invention provides the use of the method for enhancing immune response against target epitope in the preparation of a vaccine for preventing or treating diseases.

Another aspect of the present invention provides the use of mutant antigen in the preparation of a vaccine, wherein the mutant antigen is formed by incorporating a chemical cross-linking active group or a derivative thereof on target epitopes of the wild-type antigen.

Another aspect of the present invention provides a method for selecting antibodies against a specific epitope of a wild-type antigen, wherein the method comprising the following steps: (a) providing a mutant antigen, wherein the mutant antigen is incorporated with chemically cross-linked active groups or derivatives on target epitopes of the wild-type antigen; (b) administering the mutant antigen described in step (a) to the animal; (c) isolating serum from the animal; (d) selecting antibodies that specifically bind to target epitope using the wild-type antigen.

Another aspect of the present invention provides a method for selecting antibodies against a target epitope of a wild-type antigen, wherein the method comprising the following steps: (a) providing a mutant antigen, wherein the mutant antigen is incorporated with chemically cross-linked active groups or derivatives on target epitope of the wild-type antigen; (b) administering the mutant antigen described in step (a) to the animal; (c) isolating B cells from the animal, and fusing them with myeloma cells to generate hybridoma cells; (d) screening antibodies against target epitopes from culture supernatant of the hybridoma cells by using the wild-type antigen.

Another aspect of the present invention provides a method for selecting antibodies against a target epitope of a wild-type antigen, wherein the method comprising the following steps: (a) providing a mutant antigen, wherein the mutant antigen is incorporated with chemically cross-linked active groups or derivatives on target epitopes of the wild-type antigen; (b) administering the mutant antigen described in step (a) to the animal; (c) isolating B cells from the animal and applying them to construction of antibody library; (d) selecting antibodies that specifically binds to target epitope from the antibody library by using the wild-type antigen.

Another aspect of the present invention provides a method for selecting antibodies against a target epitope of a wild-type antigen, wherein the method comprising the following steps: (a) providing a mutant antigen, wherein the mutant antigen is incorporated with chemically cross-linked active groups or derivatives on target epitopes of the wild-type antigen; (b) administering the mutant antigen described in step (a) to the animal; (c) isolating serum from the animal; (d) incubating the isolated serum with the mutant antigen described in step(a) under certain conditions so that the mutant antigen and antibody are covalently cross-linked to form mutant antigen-antibody complex; (e) removing antibodies that are not covalently cross-linked with mutant antigen by certain washing conditions and releasing antibodies covalently cross-linked with the mutant antigen; (f) selecting antibodies against target epitopes from antibodies that are covalently cross-linked with the mutant antigen.

Another aspect of the present invention provides a method for selecting antibodies against a target epitope of a wild-type antigen, wherein the method comprising the following steps: (a) providing a mutant antigen, wherein the mutant antigen is incorporated with chemically cross-linked active groups or derivatives on target epitopes of the wild-type antigen; (b) administering the mutant antigen described in step (a) to the animal; (c) isolating B cells from the animal and fusing them with myeloma cells to produce hybridoma cells; (d) incubating the mutant antigen with the culture supernatant of the hybridoma cells under certain conditions so that the mutant antigen and antibody are covalently cross-linked to form mutant antigen-antibody complex; (e) removing antibodies that are not covalently cross-linked with mutant antigen by certain washing conditions and releasing antibodies covalently cross-linked with the mutant antigen; (f) selecting antibodies against target epitopes from antibodies that are covalently cross-linked with the mutant antigen.

Another aspect of the present invention provides a method for selecting antibodies against a target epitope of a wild-type antigen, wherein the method comprising the following steps: (a) providing a mutant antigen, wherein the mutant antigen is incorporated with chemically cross-linked active groups or derivatives on target epitopes of the wild-type antigen; (b) administering the mutant antigen described in step (a) to the animal; (c) isolating B cells from the animal and applying them to construction of antibody library; (d) incubating the mutant antigen with the antibody library under certain conditions, so that the mutant antigen and the antibody are covalently cross-linked to form mutant antigen-antibody complex; (e)removing antibodies that are not covalently cross-linker with mutant antigen by certain washing conditions and releasing antibodies covalently cross-linked with the mutant antigen; (f) selecting antibodies against target epitopes from antibodies that are covalently cross-linked with the mutant antigen.

In some embodiments, the chemically active group or derivative thereof is incorporated by genetic codon expansion or by chemical synthesis.

In some embodiments, the group with chemical crosslinking activity is Nε-crotonyl or Nε-acryloyl.

In some embodiments, the group with chemical crosslinking activity is a natural amino acid or a non-canonical amino acid.

In some embodiments, the non-canonical amino acid with a chemical cross-linking active group is Nε-crotonyl-L-lysine (CK) or Nε-acryloyl-L-lysine (AK).

In some embodiments, the animal is a rodent, a non-human mammal, or a mammal. In a specific embodiment, the rodent is a mouse or a rat; in a specific embodiment, the non-human mammal is a rabbit, an alpaca or a sheep, etc.; in a specific embodiment, the mammals are humans.

In some embodiments, the antigen is a soluble protein, a soluble polypeptide, a transmembrane protein expressed on a phospholipid membrane, or a polypeptide expressed on a phospholipid membrane.

In some embodiments, the conditions for incubation of the mutant antigens with the serum are alkaline conditions. In some embodiments, the conditions for incubation of the mutant antigens with the hybridoma cells are alkaline conditions. In some embodiments, the conditions for incubation of the mutant antigens with the antibody library are alkaline conditions.

In some embodiments, the alkaline condition is a solution with pH8.8; in some embodiments, the alkaline condition is a DPBS solution with pH8.8; in some embodiments, the incubation condition is incubation for 24h, 48h, 3d, 4d, 5d, 6d or 7d; In some embodiments, the incubation condition is in the DPBS solution with pH8.8, and the incubation time is 24h, 48h, 3d, 4d, 5d, 6d or 7d; In some embodiments, the incubation condition is incubation in a DPBS solution with pH 8.8 for 24 hours; in some embodiments, the incubation condition is incubation in a DPBS solution with pH 8.8 for 48 hours.

In some embodiments, the elution conditions for removing antibodies that are not covalently cross-linked to the mutant antigen are: i) alkaline elution with a high pH buffer; ii) low pH elution buffer for acidic elution.

In some embodiments, the release of the antibody from the covalently cross-linked antibody-mutant antigen complex is carried out by enzymatic digestion.

Another aspect of the present invention provides the use of the method for selection of antibodies against target epitopes of wild-type antigens in preparation of vaccines for preventing or treating diseases.

Another aspect of the present invention provides a method for preparing an antibody, comprising obtaining an antibody by the method for selecting antibodies against a target epitope of a wild-type antigen as provided herein; and providing the antibody thus obtained.

In some embodiments, the antibody obtained by selecting against the target epitope of the wild-type antigen IL-1β. In some embodiments, the antibody specifically binding to IL-1β has: the VH shown in SEQ ID NO.6 and the VL shown in SEQ ID NO.4; the VH shown in SEQ ID NO.10 and VL shown in SEQ ID NO.8; VH shown in SEQ ID NO. 14 and VL shown in SEQ ID NO. 12; VH shown in SEQ ID NO. 18 and SEQ ID NO. 16 VL; VH shown in SEQ ID NO.22 and VL shown in SEQ ID NO.20; VH shown in SEQ ID NO.26 and VL shown in SEQ ID NO.24; or VH shown in SEQ ID NO.30 and VL shown in SEQ ID NO.28.

Another aspect of the present invention provides the use of the antibody specifically binding to IL-1β in the preparation of a medicament for the treatment and prevention of IL-1-mediated diseases; wherein the IL-1-mediated diseases include adult sti Dyer's disease, systemic juvenile idiopathic arthritis, osteoarthritis, rheumatoid arthritis, gouty arthritis, acute gout, multi-system inflammatory disease of the newborn, Behcet's Disease, cryopyrin associated periodic syndrome, Familial Mediterranean fever, Hereditary periodic fever, Periodic fever syndrome, TNFR-associated periodic fever syndrome, Atherosclerosis, Atrial fibrillation, Acute myocardial infarction, Peripheral arterial disease, Chronic idiopathic Urticaria, abdominal aortic aneurysm, colorectal cancer, triple negative breast cancer, non-small cell lung cancer, type 1 diabetes, type 2 diabetes, mevalonate kinase deficiency, Schnitzler Syndrome; Urticaria and macroglobulinemia, sickle cell anemia, pyoderma gangrenosum, chronic obstructive pulmonary disease, dry eye, pulmonary sarcoidosis, Kawasaki disease, wet age-related macular degeneration, scleritis, grapevine meningitis, Muckle-Wells Syndrome, acne vulgaris, pyoderma gangrenosum, etc.

Another aspect of the present invention provides the use of the mutant antigen in preparing a vaccine for preventing or treating diseases. In some embodiments, the mutant antigen is the novel corona virus S protein RBD with AK incorporation on the K417 epitope, Y453 epitope, E484 epitope or N501 epitope; in some embodiments, the mutant antigen is is the novel corona virus S protein RBD with CK incorporation on the K417 epitope, L452 epitope, Y453 epitope, E484 epitope or N501 epitope.

### Brief Description of the Drawings

Figure 1 shows the serum antibody titer of immunized mice.
Figure 2A is the WB results of CL-E2 phage binding with WT IL-1β and hIL-1βE64AK; Figure 2B and C are the WB results of CL-E2-mFc fusion protein binding with WT IL-1β and its mutants; Figure 2D is the WB results of canakinumab binding with hIL-1βE64AK and IL-1βE64CK under chemical cross-linking conditions.
Figure 3A is the ELISA results of CL-E2-mFc binding with hIL-1β and hIL-1βE64AK, with Kd 3.7±0.2nM and 4.2±0.2nM for CL-E2-mFc binding to hIL-1β and hIL-1βE64AK, respectively; 3B is the ELISA result of CL-E2 phage binding to hIL-1β and hIL-1βE64AK, where the abscissa is the pfu value of the phage.
Figure 4A is the ELISA result of the panned phage clones binding with WT hIL-1β and hIL-1βE64AK;
Figure 4B is the ELISA result of E64AK-A9-mFc antibody fusion protein binding with WT hIL-1β and hIL-1βE64AK (with Kd value 1.6±0.2nM and 1.2±0.2nM respectively); Figure 4C and D are the WB results of E64AK-A9-mFc antibody fusion protein binding with hIL-1βE64AK, wherein the antibody used in Figure 4C is mouse anti-His-tag antibody , and the antibody used in Figure 4D is anti-mouse Fc.
Figure 5 shows the ELISA results of different phage clones binding with hIL-1βE64AK and hIL-1β63-66A, where * means p<0.01, ** means p<0.01, *** means p<0.001, **** means p<0.0001.
Figure 6A is the ELISA results of Gevokizumab binding with hIL-1β and hIL-1β63-66A, and Figure 6B is the ELISA results of E64AK-F4-mFc binding with hIL-1β or its mutants; Figure 6C and D are ELISA results of competitive inhibition of canakinumab on binding of E64AK-F4 phage or E64AK-F4-mFc with hIL-1β.
Figure 7A is the ELISA results of different phage clones binding with hIL-1β and hIL-1β64CK; Figure 7B and 7C are the ELISA results of different phage clones binding with hIL-1βE64CK and hIL-1β63-66A;
Figure 7D is the ELISA results of competitive inhibition of E64AK-A9-mFc on binding of E64CK-H11 with hIL-1β; Figure 7E is the ELISA results of competitive inhibition of E64CK-B9-mF on binding of E64AK-G6 or E64AK-A2 with hIL-1β, where, * means p<0.01, ** means p<0.01, *** means p<0.001, **** means p<0.0001.
Figures 8A and 8B are the ELISA results of antibody fusion proteins E64CK-A5-mFc, E64CK-G9-mFc binding with hIL-1β or its mutant proteins; Figure 8C and D are the ELISA results of competitive inhibition of canakinumab on the binding of antibody fusion proteins E64CK-A5-mFc or E64CK-G9-mFc with hIL-1β;
Figure 8E and 8F are the ELISA results of competitive inhibition of canakinumab on the binding of phage clones E64CK-A59 or E64CK-G9 with hIL-1β; Figure 8G is the WB results of antibody fusion proteins E64CK-A5-mFc, E64CK-G9-mFc binding with IL-1β or its mutant proteins incubated at pH 8.8, 37°C for 48 hours; Figure 8H shows the WB results of antibody fusion proteins E64CK-A5-mFc, E64CK-G9-mFc binding with hIL-1β or its mutant protein at pH 8.8 and 37°C for different time; * means p<0.01, ** means p<0.01, *** means p<0.001,* *** indicates p<0.0001.
Figure 9 shows the ELISA results of hIL-1β or hIL-1β63-66A binding with clones selected from the phage library constructed from hIL-1β-immunized mice.
Figure 10 shows the ELISA results of hIL-1βE64BK or hIL-1β63-66A binding with clones selected from the phage library constructed from hIL-1βE64BK-immunized mice, where **** indicates p<0.0001.
Figure 11A is the SDS-PAGE result while Figure 11B and Figure 11C are the ELISA results of canakinumab and gevokizumab binding with WT hIL-1β or hIL-1βQ15G, respectively.
Figure 12 is the ELISA result of the clones selected from the phage library constructed from hIL-1βQ15CK immunized mice binding with hIL-1β or its mutants, where ** means p<0.01, *** means p<0.001, **** indicates p<0.0001.
Figure 13 shows the difference in the affinity of Q15CK-G8-mFc binding with hIL-1β and its mutants as detected by ELISA.
Fig. 14A is the serum titer of mice immunized with hIL-1β and its mutants (incorporation of CK or pNO2F at different sites); 14B is the neutralization result of serum IgG neutralization experiment from hIL-1β and its mutants immunized mice; 14C HEK-Blue IL-1R inhibition results for serum IgG from hIL-1βQ15CK immunized mice.

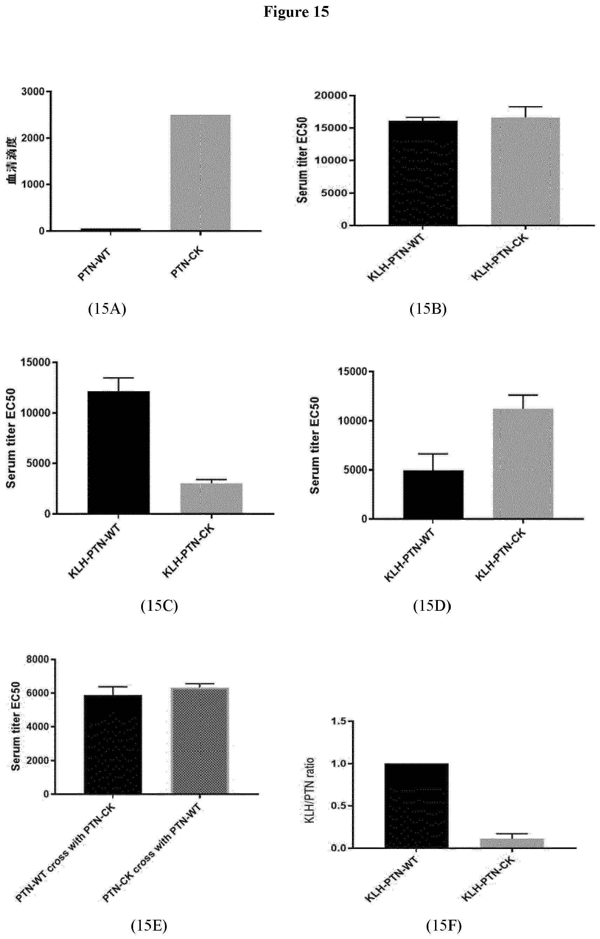

Figure 15 is the titer of different immune sera against different proteins, wherein 15A is the titer of two groups of (PTN-WT, PTN-CK) immunized mouse serum against respective immune antigens; 15B is the titer of two groups of (KLH-PTN-WT, PTN-CK) KLH-PTN-CK) immune serum titers to their respective immune antigens; 15C is the titer of two groups (KLH-PTN-WT, KLH-PTN-CK) immune serum to KLH protein; 15D is the titer of two groups (KLH-PTN -WT, HLH-PTN-CK) immune serum to the titers of respective immune polypeptides (respectively being PTN-WT, PTN-CK); 15E is KLH-PTN-CK immune serum to PTN-WT, and KLH-PTN-WT The titer of immune serum to PTN-CK; the titer ratio of immune serum to KLH/PTN in the 15F two groups (KLH-PTN-WT, KLH-PTN-CK).
Figure 16 is the analysis of different subtypes of IgG against KLH (16A) and PTN polypeptide (16B) in the immune sera of two groups (KLH-PTN-WT, KLH-PTN-CK).

### Detailed Description of the Embodiments

The invention is described in detail herein by reference using the following definitions and examples. The contents of all patents and publications mentioned herein, including all sequences disclosed in such patents and publications, are expressly incorporated herein by reference.

As used herein, the term "chemically cross-linking active group" refers to a chemical group that can covalently cross-link with amino acid residues of adjacent protein under suitable conditions. "Chemical cross-linking active groups" may include natural amino acids, derivatives of natural amino acids, and non-canonical amino acids. Non-limiting examples of "chemically cross-linking reactive groups" include Nε-crotonyl, Nε-acryloyl or p-acrylamide groups. Non-limiting examples of "non-canonical amino acids with chemical cross-linking active groups" include Nε-crotonyl-L-lysine (Nε-crotonyl-L-lysine, CK), Nε-acryloyl-L-lysine acid (Nε-acryloyl-L-lysine, AK), p-acrylamido-(S)-phenylalanine (p-acrylamido-(S)-phenylalanine), natural amino acids with or incorporated with nucleophilic groups (for example, lysine with ε-amino group) and the like.

As used herein, the term "chemical cross-linking" means that a group having chemical cross-linking activity can covalently cross-link with a group of amino acid residues adjacent to a protein under suitable conditions to form a complex.

As used herein, the term "non-canonical amino acid" refers to an amino acid that is not one of the 20 classical amino acids or selenocysteine or pyrrolysine. Other terms that may be used synonymously with the term "non-canonical amino acid" are "non-naturally encoded amino acid", "unnatural amino acid", "non-naturally occurring amino acid". The term "non-canonical amino acid" also includes, but is not limited to, amino acids that have been modified (e.g., post-translationally) by naturally encoded amino acids, including but not limited to the 20 common amino acids or pyrrolysine and selenocysteine, but are not themselves naturally incorporated into the polypeptide chain by the translation complex. "Non-canonical amino acids" can include various functional groups or reactive groups, which can provide additional functions and/or activities.

As used herein, the term "mutant antigen" refers to an antigen formed by incorporating a chemical cross-linking active group or a derivative thereof into the target epitope of the wild-type antigen. The term "wild-type antigen" includes not only soluble proteins and soluble polypeptides, but also transmembrane proteins or polypeptides expressed on phospholipid membrane structures; "wild-type antigens" can be derived from animals, plants or microorganisms (such as bacteria, fungi, viruses).

As used herein, the term "vaccine" refers to an antigen that induces an organism to produce antibodies against an epitope of interest. Antigens that enhance an organism's immune response to a specific epitope to target antigen are also included in the present invention. A non-limiting example of the vaccine of the present invention includes a mutant antigen formed by incorporating a group with chemical cross-linking activity or a derivative thereof into the target epitope of the wild-type antigen.

### EXAMPLES

### 1. Expression and purification of WT IL-1β and its mutants

In order to overexpress WT hIL-1β, IL-1β single alanine mutant (hIL-1βE64A) and IL-1β mutant containing four alanine mutations (hIL-1β63-66A), The pET28a expression vector containing the gene sequence above was transformed into Escherichia coli BL21(DE3) competent cells. Clones are picked and inoculates into 500ml of 2 × YT medium containing kanamycin (50µg/ml), and then cultured at 37°C. When the OD600 reaches 0.6, 0.5mM isopropyl-β-D-thiogalactopyr Pyranoside (IPTG) was added and inducted at 30°C overnight. To overexpress AK- or CK-incorporated hIL-1β mutants, pEVOL-MmAKRS or pEVOL-MmCKRS vector is co-transformed with amber codon (TAG)-containing hIL-1β expressing vector into BL21(DE3) competent cells. Clones were picked and inoculated into 2 × YT medium containing kanamycin (50 µg/ml) and chloramphenicol (25 µg/ml), then cultured for about 3-5 hours. When the OD600 reaches 0.8, 1mM IPTG, 5mM CK or 10mM AK was added, and L-arabinose (m/v) with a final concentration of 0.2% was further added to induce the expression of UAA-incorporated protein. For preparation of BK or pNO2F-incorporated proteins, the corresponding orthogonal plasmids pUltra-pNO2RS (Tsao et al., 2006) or pDule-pylRS (Lang and Chin, 2014) was applied and the rest of the preparing steps were the same as for the CK-incorporated mutants. The culture was grown at 30°C for 15 hours, harvested by centrifugation at 6,000g for 10 minutes, lysed by sonication, and centrifuged at 13,000g for 30 minutes at 4°C for collection of the supernatant of the cell lysate. WT hIL-1β and mutants were purified on Ni-NTA resin (GE Healthcare, 17-0575-01) following the manufacturer's instructions. The protein was further purified in DPBS buffer by Superdex 200increase10/300GL column (GE Healthcare, 10263259) and stored at -80°C.

### 2. Mice immunization

WT hIL-1β or IL-1β mutants with incorporation of non-canonical amino acids (hIL-1βE64AK, hIL-1βE64CK, hIL-1βE64BK, hIL-1βQ15CK) were injected subcutaneously into 6-8 week-old female Balb/C mice (3 per group). For the first immunization, 50 µg of antigen was mixed with Freund's complete adjuvant (sigma, F5881) before injection, and for the second and third immunization, 30 µg of antigen was mixed with Freund's incomplete adjuvant (sigma, F5506) before injection. The interval between two immunizations was 2 weeks.

### 3. Mouse total IgG Purification

After three immunizations, mouse serum was collected and diluted with an equal volume of DPBS (pH 8.0). The sample was then incubated with protein A resin (GenScript, L00210) for 3 hours, and after washing with DPBS of 10 times the column volume, the protein bound to protein A was eluted with elution buffer (0.2M glycine, 0.1M NaCl, pH 2.5). Immediately after elution, Tris-HCl (final concentration 100 mM) was added to adjust the pH to 7.5. Then Amicon Ultra spin column (Merck Millipore, UFC903096) was used to concentrate and exchange medium (DPBS, pH 7.5).

### 4. Phage library construction

Phage display libraries were constructed using published methods (Barbas et al., 1991). To construct the mouse immune library, Balb/c mice were immunized three times with wild-type hIL-1β, hIL-1βE64AK, hIL-1βE64CK, hIL-1βE64BK or hIL-1βQ15CK, respectively, with an interval of 2 weeks between two immunizations. Two weeks after the third immunization, the total RNA of mouse spleen was extracted and used as a template for reverse transcription to construct a cDNA library. The scFv phage display library was constructed using the phagemid vector pSEXRTL2, and the M13KO7 (ΔpIII) helper phage (PROGEN, catalog number: PRHYPE) was further used to package the library into scFv-pIII phage.

### 5. Phage Production

Escherichia coli XL1-Blue cells carrying phagemids (displaying scFv-pIII) were inoculated into 20 ml of 2 × YT medium, ampicillin (100 µg/ml) and tetracycline (15 µg/ml) were added and incubated at 37 ° C, 220rpm. When OD600 reached 0.5, M13KO7(ΔpIII) helper phage with multiplicity of infection (MOI)=20 was added and incubated at 37°C and 120rpm for 1 hour. Then the culture was centrifuged, and the precipitate was resuspended in 40ml of 2 × YT medium (100µg/ ml ampicillin, 15 µg/ml tetracycline and 50 µg/ml kanamycin) at 30° C., 250 rpm for 13 hours. The culture was further centrifuged at 4000g for 10 minutes and the supernatant was transferred to a new tube and centrifuged at 10,000g for 20 minutes to remove cell debris. 5X phage precipitation buffer [100g PEG 8000, 73.3g NaCl dissolved in 500ml ddHO] was added and incubated on ice for 4 hours. Phages were collected by centrifugation at 10,000g for 20 minutes at 4°C, dissolved in 1ml DPBS, and incubated at room temperature for 15 minutes. The phage was filtered with a 0.22 µm filter membrane and stored at 4°C for future use.

### 6. Phage Panning

Conventional phage panning: WT IL-1β antigen (1 µg) was coated in wells in DPBS at 4°C overnight, then blocked with 200 µl of DBPS containing 3% non-fat dry milk for 2 hours at room temperature. After washing twice with DPBST, 10E0 pfu of phage obtained from the library constructed from immunizing mice with wild-type hIL-1β, hIL-1βE64AK, hIL-1βE64CK, hIL-1βE64BK or hIL-1βQ15CK was added and incubated at room temperature for 2 hours. After washing with DPBST 10 times (3 minutes each time), 1 mg/ml trypsin (Gibco) was added to digest and recover the antigen-bound phages.
Chemically cross-linked phage panning: hIL-1βE64AK (1 µg) was coated in a 96-well plate at 4°C overnight, blocked with 200 µl DPBS containing 3% BSA for 2 hours at room temperature. 10E10 pfu of phage (containing 1% BSA, 1 mM EDTA, pH 8.8) obtained from hIL-1βE64AK immune phage library was added and incubated at 37°C for 48 hours. Wash wells under stringent conditions, including: 2 times (5 minutes in total) washing with DPBS containing 10mM DTT; 10 times (20 minutes in total) washing with DPBST; 2 times (3 minutes in total) washing with 0.15% SDS solution; 10 times (total 20 minutes) washing with DPBS; once washing (total 3 minutes) with acidic buffer (0.2M glycine, pH 2.2); 10 times (total 20 minutes) washing with DPBST; twice (total 5 minutes) washing DPBS. After washing, 1 mg/ml trypsin (Gibco) was added and incubated for 20 min to release the antigen-bound phage. The collected phages were used for Escherichia coli XL1-Blue infection to produce phages.
Positive clones after panning were randomly selected for sequencing and homology analysis. Using ClustalW (MEGA-X; DNA Weight Matrix: IUB; Gap opening penalty: 15.00; Gap Extension penalty: 6.66) for sequence alignment of all scFv, and calculate the maximum likelihood tree.

### 7. Construction and expression of fusion protein of scfv from example 6 with mFc

The scfv fragment of the positive clone CL-E2, E64AK-A9, E64AK-F4, E64CK-B9, E64CK-A5, E64CK-G9 or Q15CK-G8 obtained by panning in example 6 was fused to the Fc of mouse IgG2a by a linker (named as CL-E2-mFc, E64AK-A9-mFc, E64AK-F4-mFc, E64CK-B9-mFc, E64CK-A5-mFc, E64CK-G9-mFc, or Q15CK-G8-mFc, respectively), and then cloned into pFuse vector for expression. HEK 293F cells (Thermo Scientific, R79007) were cultured, and the scFv-mFc expression plasmid constructed above were transfected into cells (2.5 × 10 6 cells/ml) with the help of PEI at a ratio of 1:2.5 (mass ratio). When the cell viability dropped below 75%, the cell culture supernatant was collected, passed through protein A resin (GenScript, L00210) pre-equilibrated in DPBS twice, washed with 10 times column volume of DPBS, and eluted with Buffer (0.2M Glycine, 0.1M NaCl, pH 2.5). Immediately after elution, Tris-HCl (final concentration 100 mM) was added to adjust the pH to 7.5. Then Amicon Ultra spin column (Merck Millipore, UFC903096) was used to concentrate and change medium (DPBS, pH 7.5), and SEC purification (chromatographic column: Superdex 200 increase 10/300 GL, GE Healthcare, 10263259) was applied.

### 8.ELISA

Antigen (100 ng) was coated on 96-well ELISA plate (Corning Costar, 2592) overnight at 4°C and blocked with 200 µl of DPBS containing 3% skim milk powder for 2 hours at 37°C. Antibody or phage in DPBST solution containing 3% skimmed milk powder was added and incubate at 37°C for 2 hours. After washing four times with 200 µl DPBST, horseradish peroxidase (HRP)-conjugated detection antibody was added and incubated at room temperature for 1 hour. After washing with 200 µl DPBST five times, 100 µl TMB (Biolegend, 002023) chromogenic reagent was added and incubated at room temperature for 10-30 minutes. Microplate reader (BMG LABTECH, CLARIOstar^{®}Plus) was used to read the value.

**Competitive ELISA:** 100ng WT hIL-1β was coated on the ELSIA plate overnight at 4°C, blocked in DPBS containing 3% BSA at 37°C for 2 hours, then serially diluted canakinumab (DPBST containing 3% BSA) was added and incubated at room temperature for 1 hour. After incubation, 100 nM E64AK-F4-mFc, 0.2 nM E64CK-A5-mFc or 10 nM E64CK-G9-mFc obtained in example 7 were added, and incubated at room temperature for 1 hour. After washing, add HRP-conjugated goat anti-mouse IgG Fc (1:5000) and incubate at room temperature for 1 hour. TMB chromogenic reagent (Biolegend, 002023) was added and incubated for 10-30 minutes at room temperature, Microplate reader (BMG LABTECH, CLARIOstar^{®}Plus) was applied to read the value.

ELISA data were compared by two-way ANOVA analysis followed by multiple comparisons using Prism 6.0 (GraphPad software). All P values were calculated using GraphPad Prism 6.0 with the following meanings: n.s.p>0.05; *p<0.05; **p<0.01; ***p<0.001; ****p<0.0001. Details of the statistical analysis for each experiment can be found in the figures and legends.

### 9. Competitive Phage ELISA

100ng WT hIL-1β was coated on the ELSIA plate overnight at 4°C, and after blocking for 2 hours in DPBS containing 3% BSA, serially diluted phage (starting from 10 8 pfu, 10-fold dilution) in DPBST containing 3% BSA was added and incubated at room temperature for 2 hours, followed by 300 nM canakimab addition and further incubate for another 1 hour. After washing, HRP-conjugated mouse anti-M13 (antibody) (1:2000) was added and incubated at room temperature for 1 hour. After adding 100 µl TMB chromogenic reagent (TMB; Biolegend, 002023) and incubating at room temperature for 10-30 minutes, microplate reader (BMG LABTECH, CLARIOstar^{®}Plus) was to read the value. ELISA data were compared by two-way ANOVA analysis followed by multiple comparisons using Prism 6.0 (GraphPad software). All P values were calculated using GraphPad Prism 6.0 and have the following meanings: nsp>0.05; *p<0.05; **p<0.01; ***p<0.001; ****p<0.0001. Details of the statistical analysis for each experiment can be found in the figures and legends.

### 10. Western blot

Samples were mixed with loading buffer containing 20mM DTT and 2% SDS, heated at 95°C for 10 minutes, run an SDS-PAGE gel, and then electrotransfered to a PVDF membrane (Bio-Rad, 1620177). After blocking in DPBS of 5% skimmed milk powder for 2 hours, antibody was added and incubated for 2 hours. After washing with DPBST four times, HRP-labeled secondary antibody (1:5000) was added and incubated at room temperature for 1 hour, and further washed with DPBST for 4 times. ECL reagent (Thermo Fisher Scientific, 35055) was applied for color development, and the image was read on Tanon 5200.

### 11. Chemical cross-linking reaction

8 µM hIL-1βE64AK, hIL-1βQ15AK, hIL-1βE64CK or hIL-1βQ15CK with 4 µM corresponding antibodies (CL-E2-mFc, E64AK-A9-mFc, E64CK-A5-mFc and E64CK-G9-mFc) were incubated in alkaline DPBS (containing 1 mM EDTA, pH 8.8) at 37°C for 2 days (48h) or 5 days. For phage chemical cross-linking reaction, 10E8 pfu phage (CL-E2) were incubated with 8 µM hIL-1βE64AK under the same alkaline conditions for 2 days. All reactions were performed under sterile conditions.

### 12. hIL-1β neutralization experiment

HEK-Blue TM IL-1R cells (Invivogen, hkb-il1r) with a density of 70% were washed twice with pre-warmed PBS, and then resuspended in pre-warmed DMEM medium (containing 10% heat-inactivated FBS) (cell density 330,000 cells/ml medium). In a separate 96-well, 25 µl of recombinant human IL-1β (0.8 ng/ml) was incubated with 25 µl serially diluted (1:5) purified serum IgG (initial concentration 4 µM) for 30 minutes at room temperature. 150 µl of HEK-Blue IL-1R cell suspension (approximately 50,000 cells) was added to each well. The 96-well cell culture plate was incubated overnight in a 5% CO2, 37°C cell culture incubator. 20 µl of cell culture supernatant was taken and incubated with 180 µl of QUANTI-Blue TM (Invivogen) at 37° C for 30 minutes to 3 hours. Secreted embryonic alkaline phosphatase (SEAP) was detected at 655nm by a microplate reader (CLARIOstar^{®}Plus).

### Experimental results

### 1. AK-incorporated hIL-1β can induce production of antibodies with cross-linking activity

AK is a lysine-derived nocanonical amino acid whose acrylamide group on the side chain can form a covalent bond with adjacent nucleophilic group (Furman et al., 2014). We speculate that after immunization with AK-incorporated antigens, antibodies that can covalently cross-link with AK-incorporated antigens be evoked duing B cell hypermutation in mice.

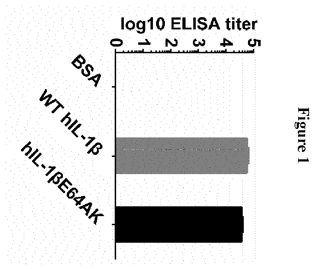

We selected human IL-1β to verify our speculation. Basing on the crystal structure of hIL-1β-canakinumab (Fab) complex (PDB: 4G6J), E64, the key binding site of hIL-1β and canakinumab, was selected for AK incorporation. pEvol vector encoding MmAKRS/tRNA CUA orthogonal pair and pET28a-hIL-1βE64TAG were co-transformed into E.coli BL21(DE3), and the hIL-1βE64AK mutant protein was purified by nickel column and size exclusion chromatography (SEC). Balb/ c mice were immunized with hIL-1βE64AK mutant protein after identification by ESI-MS mass spectrometry. After three immunizations, the serum titer was detected by enzyme-linked immunosorbent assay (ELISA). Results showed the similar potency (approximately 1: 10E5) of mouse serum binding to WT hIL-1β and IL-1βE64AK (FIG. 1A). Total RNAs were isolated from mouse spleen, reversely transcribed into cDNA library, and applied to construct scfv phage library (Barbas et al., 1991).

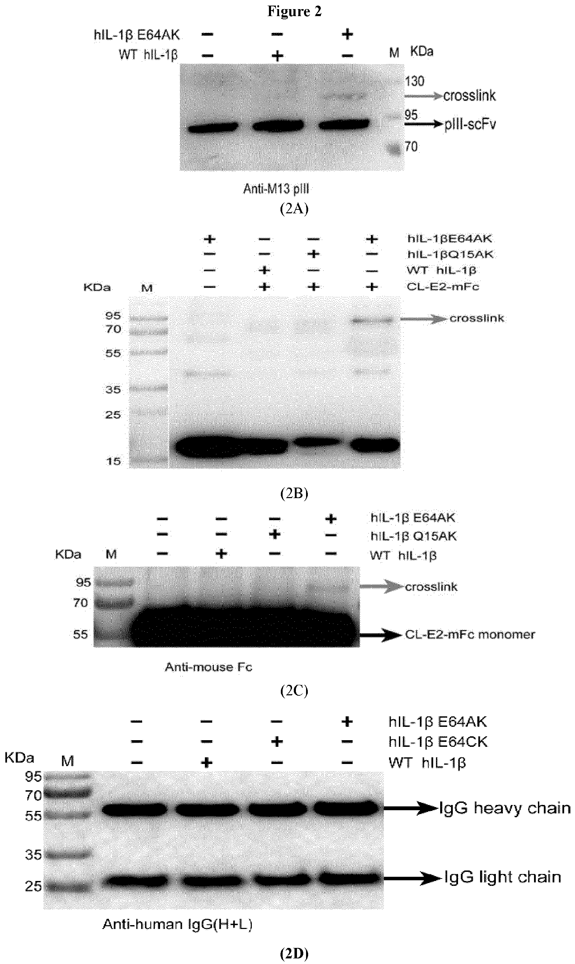

Next, chemical cross-linking panning on hIL-1βE64AK immune phage library was performed: hIL-1βE64AK (1ug/well) was coated in 96-well plate, incubated overnight at 4°C, and blocked by DPBST containing 3% BSA (0.5% Tween-20, DPBS) at room temperature for 3 hours. After washing with DPBS (pH7.5), phage library (10E10 pfu) (DPBS, pH8.8) was added and incubated at 37°C for 48 hours, then elution was perfromed as follows: 1) twice washing with DPBS containing 10mM DTT; 2) 10 times washing with DPBST; 3) twice washing with 0.15% SDS; 4) 10 times washing with DBPS; 5) once washing with glycine solution (pH 2.2); 6)10 times washing with DPBST and 2 times washing with DPBS, and finally digested by trypsin. After two rounds of chemical cross-linking panning, 28 output-positive clones were randomly selected for sequencing, and sequence homology analysis showed that 17 clones had the same amino acid sequence. One of the clones (named CL-E2) was selected for packaging monoclonal phage antibodies. After incubation of CL-E2 phage and hIL-1βE64AK in DPBS (pH8.8) for 48 hours, WB detection revealed a specific band whose size matched the size of the hIL-1βE64AK+pIII-scfv complex (Fig. 2A).

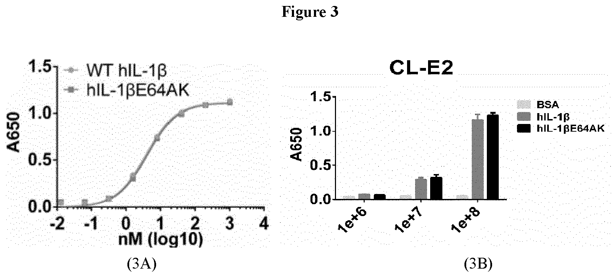

To further prove whether the binding between scfv and hIL-1βE64AK is covalent, CL-E2 was fused with the Fc of mouse IgG2a to construct CL-E2-mFc antibody, then CL-E2-mFc was incubated under the same conditions as that for chemically cross-linked. WB was performed using anti-his-tag (Figure 2B) or anti-Fc antibody (Figure 2C), and cross-linked bands was observed. However, under the same conditions, cross-linking of CL-E2-mFc with WT IL-1β was not observed. Furthermore, cross-linking of CL-E2-mFc with hIL-1βQ15AK (incorporation of AK into hIL-1βQ15) was also not observed (Fig. 2B). Canakinumab which binds to the E64 epitope did not cross-link with hIL-1βE64AK under the aforementioned chemical cross-linking conditions (Fig. 2D). Based on the above results, it can be seen that the cross-linking reaction of antigen and antibody is antibody sequence-specific and epitope specific. CL-E2 and CL-E2-mFc have similar binding affinity to WT IL-1β or hIL-1βE64AK (Figure 3A and 3B), probably B cells that could covalently cross-link with hIL-1βE64AK has been promoted during the selection of B cell clones. The covalent binding leads to proliferation of the hIL-1βE64AK-reconigzing B cells, thereby enriching a large number of hIL-1βE64AK-specific CL-E2 antibody, which was then selected through specific phage chemical panning method described above by us.

| | Nucleotide (SEQ ID NO.) | Amino acid (SEQ ID NO.) |
|---|---|---|
| hIL-1β | 1 | 2 |
| CL-E2 VL | 3 | 4 |
| CL-E2 VH | 5 | 6 |
| E64AK-A9 VL | 7 | 8 |
| E64AK-A9 VH | 9 | 10 |
| E64AK-F4 VL | 11 | 12 |
| E64AK-F4 VH | 13 | 14 |
| E64CK-B9 VL | 15 | 16 |
| E64CK-B9 VH | 17 | 18 |
| E64CK-G9 VL | 19 | 20 |
| E64CK-G9 VH | 21 | 22 |
| E64CK-A5 VL | 23 | 24 |
| E64CK-A5 VH | 25 | 26 |
| Q15CK-G8 VL | 27 | 28 |
| Q15CK-G8 VH | 29 | 30 |
| mFc | 31 | 32 |

### 2. Antigens with AK incorporated can induce epitope-directed antibody responses

AK-incorporated antigens can induce the production of antibodies with chemical cross-linking activity, and we speculate that this unique mechanism can be used for epitope-directed enrichment of antibodies that bind to AK-incorporated epitopes. To test this possibility, we assessed the abundance of antibodies binding to hIL-1βE64 epitope in hIL-1βE64AK-immunized phage libraries. After 3 rounds of conventional panning (affinity-based screening and conventional elution conditions), 96 clones were randomly selected from the obtained output clones, and then subjected to sequencing and sequence homology analysis. Compared with clones obtained in the first and third rounds of panning, the output clones after the second round of panning was endowed with both sequence diversity and higher affinity.

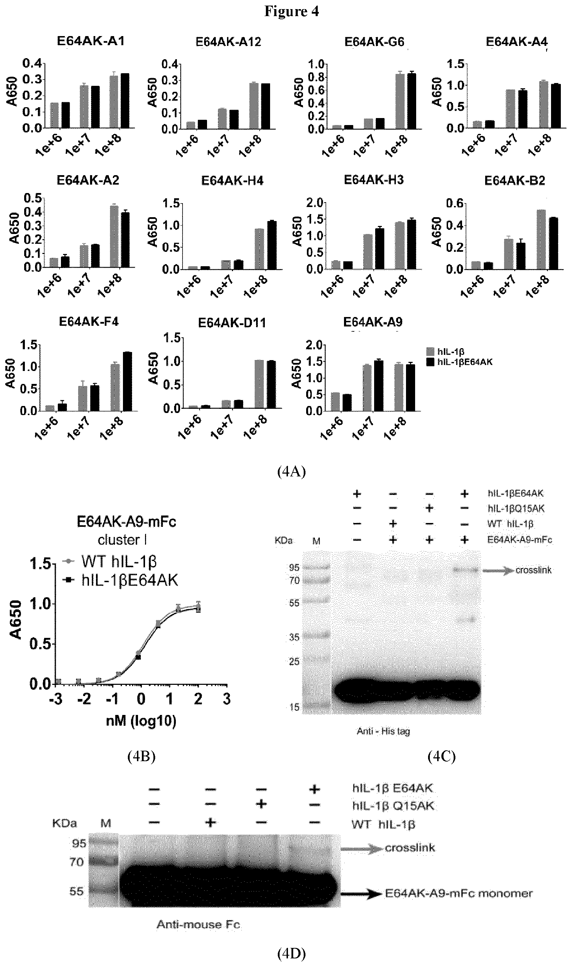

The output clones obtained in the second round of panning were applied to epitope identification. The output clones were divided into 7 clusters basing on the scfv amino acid sequence (identity<98%). Among the 80 sequences, the E64AK-A9 clone with similar affinity to WT IL-1β and hIL-1β E64AK appeared 46 times (Fig. 4A). Next, the E64AK-A9-mFc fusion antibody was constructed, and was found to have similar binding affinity to WT IL-1β (1.6±0.2nM) and hIL-1βE64AK (1.2±0.2nM) (Fig. 4B). Moreover, E64AK-A9-mFc and hIL-1βE64AK formed covalently cross-linked complexes in the aforementioned chemical cross-linking conditions (Figure 4C, 4D). Although E64AK-A9-mFc's cross-linking with hIL-1βE64AK did not appear to be particularly obvious, which may be due to fact that E64AK-A9-mFc was screened by conventional panning methods basing on non-covalent binding, these results are sufficient to suggest that chemical activity of E64AK on hIL-1β induces enrichment of antibodies that bind this epitope in its vicinity.

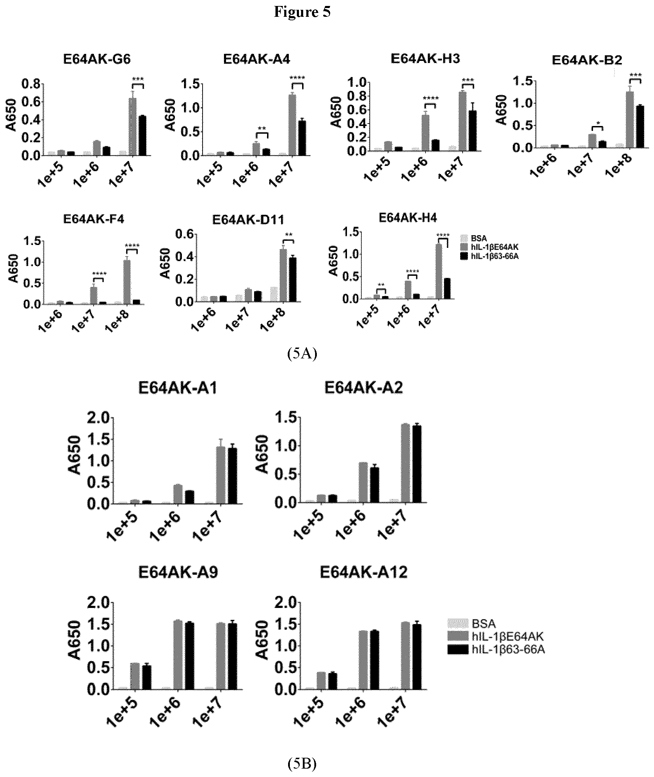

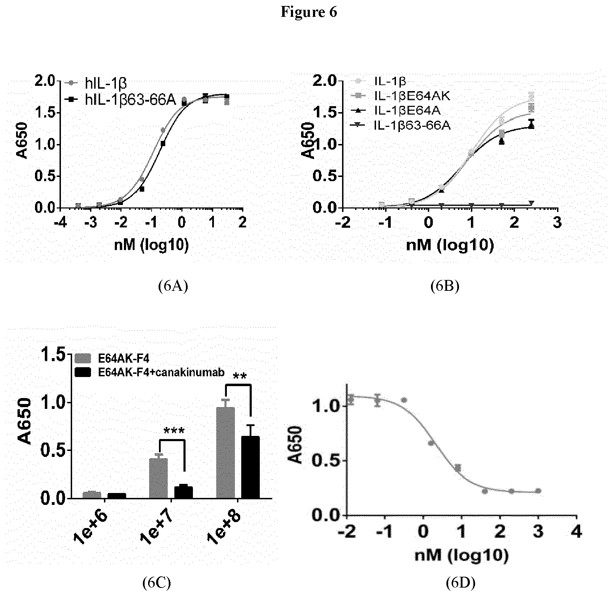

Representative scfv antibodies and three scfv antibodies that appeared only once were selected from the remaining antibody clusters and packaged into monoclonal phages. To facilitate the identification of binding epitope, IL-1β single alanine mutant (hIL-1βE64A) and IL-1β mutant containing four alanine mutations (hIL-1β63-66A) was constructed, respectively. If these phages bind to that near E64 epitope, there would be significant difference in their binding affinities with hIL-1βE64AK and the alanine mutant. The affinities of these monoclonal phages to WT hIL-1β, hIL-1βE64AK and alanine mutants were detected by ELISA, and the results showed that all 11 phages could bind to WT IL-1β (Figure 4A). With the exception of 7 monoclonal phages (E64AK-A4, E64AK-B2, E64AK-D11, E64AK-F4, E64AK-G6, E64AK-H3and E64AK-H4) which had significantly decreased affinity to hIL-1β63-66A (compared to hIL-1βE64AK) (Fig. 5A), the remaining phages bound to WT-IL-1βE64AK or hIL-1βE63-66A with similar affinities (Fig. 5B). Gevokizumab, an IL-1β high-affinity antibody that does not bind with hIL-1β63-66 epitope (Blech et al., 2013), had similar affinities to WT hIL-1β and hIL-1β63-66A (Fig. 6A), suggesting the 63-66A mutation does not have much effects on its conformation. The above results demonstrate that 7 phage antibodies with reduced affinity (compared to hIL-1βE64AK) to hIL-1β63-66A bind to hIL-1βE64 epitope. E64AK-F4 with the highest affinity difference between WT hIL-1β and hIL-1β63-66A mutants was selected to construct E64AK-F4-mFc fusion antibody. ELISA results showed that E64AK-F4-mFc binds similarly to WT hIL-1β and hIL-1βE64AK (K d =7.8±0.5 nM and 6.8±0.6 nM, respectively). However, it did not bind hIL-1β63-66A and had significantly lower affinity to hIL-1βE64A (FIG. 6B). In addition, the binding of E64AK-F4 phage and E64AK-F4-mFc fusion antibody to hIL-1β could be competitively inhibited by Canakinumab (IC 50 =2.1±0.9nM), further indicating that E64AK-F4 binds to the hIL-1βE64 epitope (Fig. 6C, D). All these experimental results showed that hIL-1βE64AK can induce antibody responses against specific epitopes.

### 3. CK-incorporated antigens can also induce epitope-specific antibodies

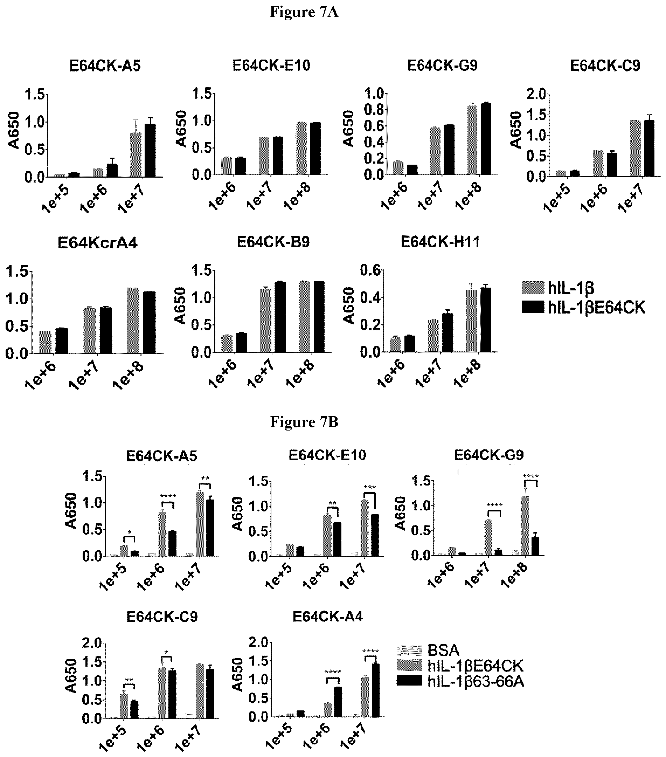

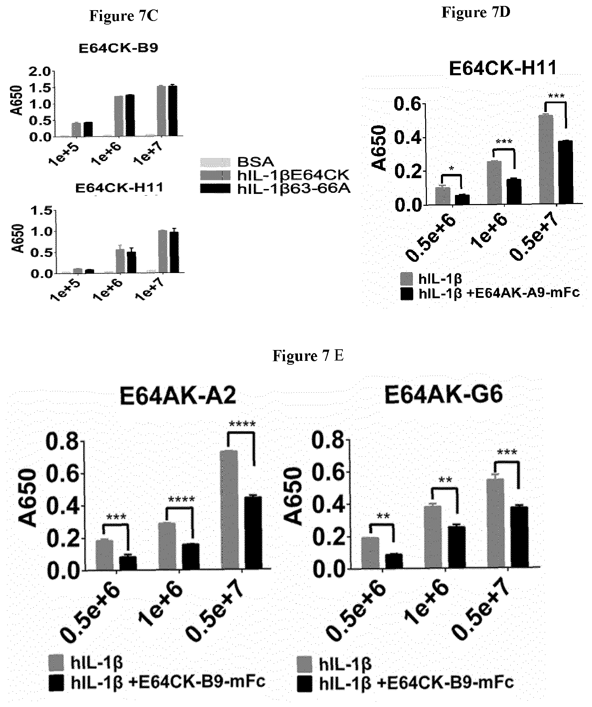

Nε-crotonyl-L-lysine (CK) is also a lysine-derived noncanical amino acid, which has weaker chemical cross-linking activity than that of AK. Inspired by the above results, we speculated that immunization of mice with CK-inserted antigens could also induce antibodies targeting specific epitopes. Antibody phage library was constructed from hIL-1βE64CK (which was expressed by genetic coding technology)-immunized mice. After 2 rounds of conventional panning, 96 output clones were randomly selected for sequencing, of which, 84 had complete mouse scfv antibody sequences. Unlike the phage antibody sequences constructed from hIL1βE64AK-immunized mice (one cluster of antibodies contained nearly half of the clones), the phage antibody sequences constructed from hIL1βE64CK-immunized mice were evenly distributed in five antibody clusters, which may be due to the weaker chemical cross-linking activity of CK. One representative sequence was selected from each cluster to package monoclonal phages, and the results showed that all of these monoclonal phages could bind to WT hIL-1β (Fig. 7A). The binding affinity of E64CK-A5/E10 (cluster 1) and E64CK-G9/C9 (cluster 2) to hIL-1β63-66A was significantly reduced when compared to hIL-1βE64CK(Fig. 7B), suggesting that these two clusters of antibodies may bind to E64 epitope of IL-1β. Interestingly, the binding affinity of E64CK-A4 with hIL-1β 63-66A was significantly higher than that for hIL-1βE64CK (Figure 7B), implying that the binding of E64CK-A4 to this epitope may not be due to its direct interaction with amino acids 63-66. However, the difference in binding affinity could also explain that this cluster antibody binds the E64 epitope of hIL-1β. E64CK-H11 and E64CK-B9, both with lower sequence homology to E64CK-A5/E10, E64CK-G9/C9, and E64CK-A4 showed no significant difference in the binding affinity with E64CK and hIL-1β63-66A (Fig. 7C). E64AK-A9 binds to the E64 epitope (Figure 4C), and the binding of E64CK-H11 to hIL-1β can be competitively inhibited by E64AK-A9-mFc (Figure 7D), suggesting that E64CK-H11 (sequence homologous to E64AK-A9) also bound the E64 epitope while its affinity was not (at least not entirely) dependent on binding to this region, explaining why there is little difference between its affinity for binding hIL-1βE64CK and hIL-1β63-66A. Likewise, since E64AK-G6 specifically bound the E64 epitope (Fig. 5A), it is assumed that E64CK-B9 should also bind the E64 epitope basing on the sequence homology between E64CK-B9 and E64AK-G6. As expected, E64CK-B9-mFc could competitively inhibit the binding of E64AK-G6 or E64AK-A2 to hIL-1β (Fig. 7E), suggesting that these clones (E64CK-B9, E64AK-G6, E64AK-A2) all bind to similar regions on hIL-1β.

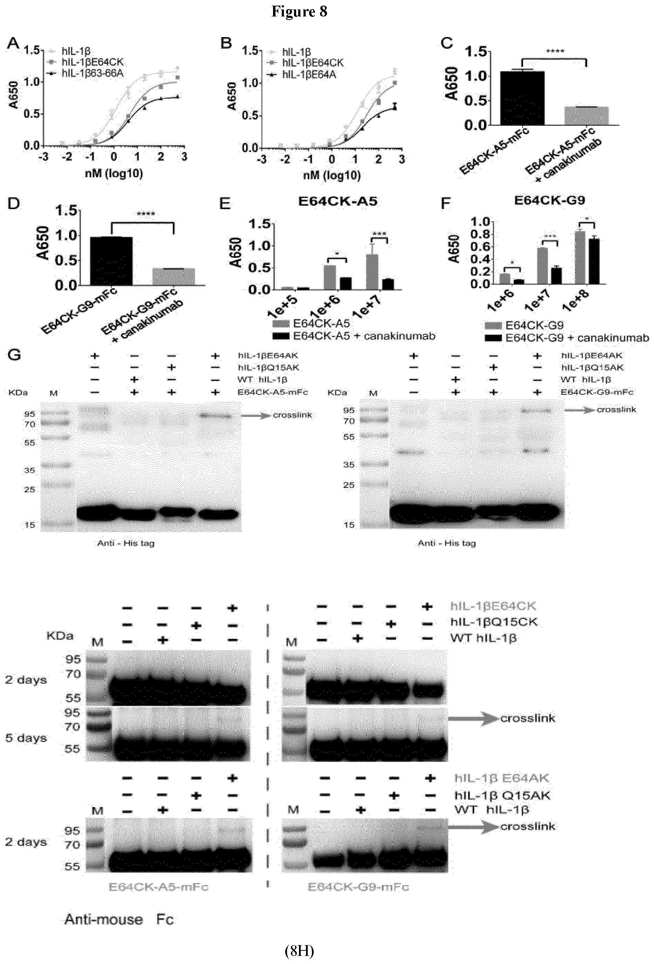

Next, E64CK-G9-mFc and E64CK-A5-mFc was expressed, and their binding epitopes at the protein level was verified. Consistent with the results of phage ELISA, the binding affinity of E64CK-A5-mFc and E64CK-G9-mFc to hIL-1β63-66A was significantly reduced (Fig. 8A, B). Moreover, canakinumab can compete with E64CK-A5-mFc and E64CK-G9-mFc for binding to hIL-1β (Fig. 8C, D), which was also consistent with the results of phage competition ELISA (Fig. 8E, F).

To investigate whether the enrichment of epitope-specific antibodies induced by hIL-1βE64CK was due to the chemical cross-linking activity of CK, E64CK-G9-mFc or E64CK-A5-mFc was incubated with hIL-1βE64CK as previously described. After incubation for 48 h under cross-linking conditions, no cross-linking complex was detected as shown by WB (Fig. 8H). However, after extending the incubation time to 5 days, a weak cross-linked band was detected (Fig. 8H). In contrast, cross-linked complex bands were detected after incubation of these two antibodies with hIL-1βE64AK for 48h (Fig. 8G,H), while incubation of these two antibodies with hIL-1βQ15AK failed to form cross-linked complex bands as detected by WB (Fig. 8G,H). Under the same cross-linking conditions, no cross-linking reaction occurred between canakinumab and hIL-1βE64CK (Fig. 2D). It can be inferred from results above that hIL-1βE64CK or hIL-1βE64AK can directly elicit immune response and enrich antibodies targeting the specific epitope by site-specific cross-linking activity during the process of clone selection and B cell hypermutation.

### 4. Epitope-specific antibody responses are due to the chemical cross-linking activity of AK or CK

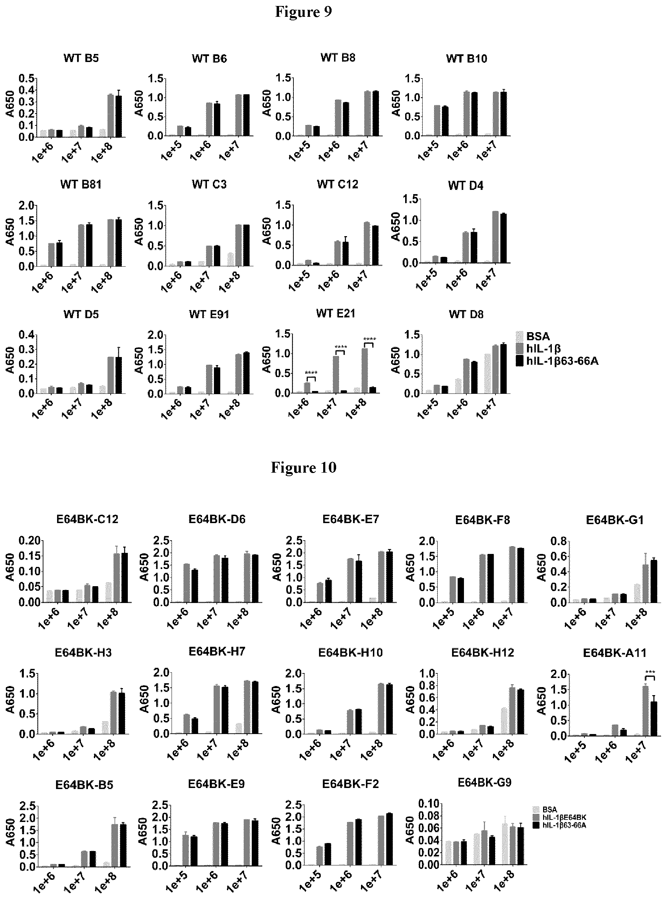

In order to rule out that the possibility that induction of antibody responses specific to hIL-1βE64CK or hIL-1βE64AK epitopes is due to the specific sequence near hIL-1βE64 epitope, mice was immunized with WT IL-1β. Then immune phage antibody library was constructed and subjected to routine panning as described previously. After two rounds of panning, 96 clones were randomly selected for sequencing, and divided into 12 clusters basing on sequence homology. Of eighty-seven clones with mouse scfv sequences, only one clone (WT E21) could bind to the E64 epitope (Fig. 9). Moreover, nocanonical amino acid Nε-Boc-L-Lysine (BK) without cross-linking activity was incorporated at the E64 position of hIL-1β, and a phage library was constructed from hIL-1βE64BK-immunized mice. After two rounds of panning, 72 clones were randomly selected for sequencing, and the results showed that only one cluster of antibodies (E64BK-A11, with frequency 2) seemed to bind hIL-1βE64 epitope (Figure 10). Taking the above results together, the epitope-directed antibody response may be due to the chemical cross-linking activity of AK and CK incorporated within the specific epitope.

### 5. CK-induced epitope-specific antibody responses are independent of epitope sequence

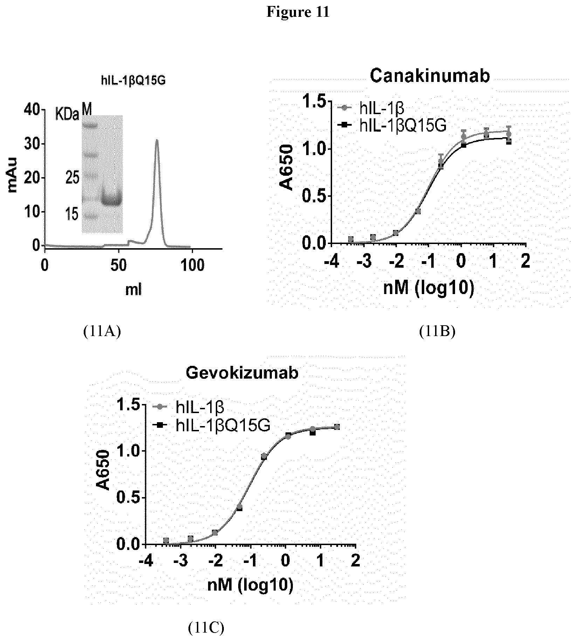

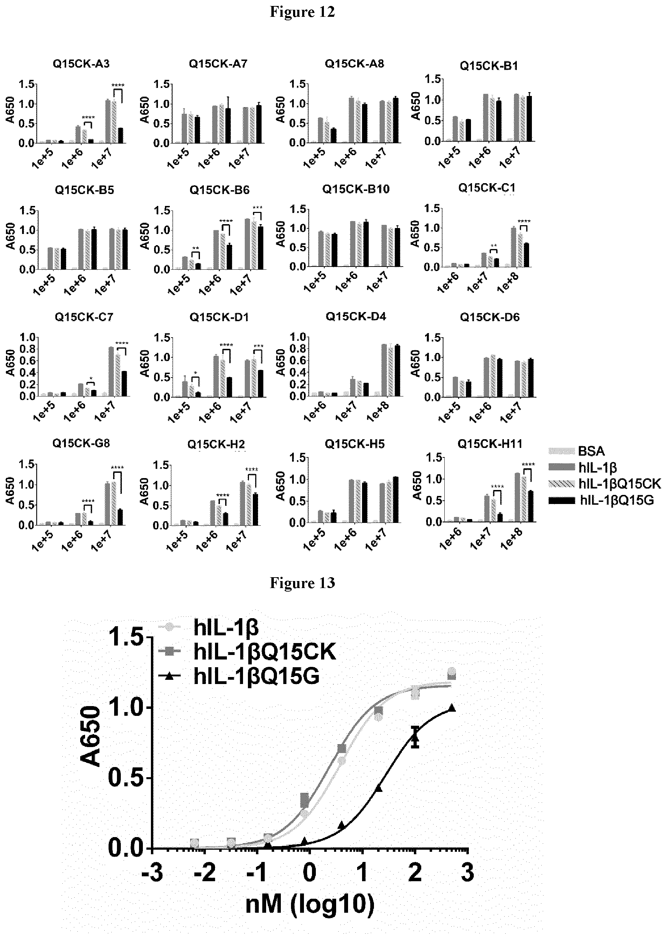

Our data show that antigens incorporated with CK or AK are effective in inducing antibody responses against epitopes near E64. To investigate whether this mechanism is independent of epitope sequence, we chose another epitope of hIL-1β for CK incorporation. Q15 is an important site for IL-1β binding to IL-1RI (Evans et al., 1995). hIL-1βQ15CK was constructed, expressed and purified according to the steps described above, and then hIL-1βQ15CK-immunized mice were subjected to constructing phage antibody library. After 2 rounds of panning, 96 clones were randomly selected for sequencing, and divided into 12 clusters basing on the homology of amino acid sequences, among which 89 output clones contained complete mouse scfv sequences. 1-2 representative clones from each antibody cluster and 3 clones not in the antibody cluster were subjected to package into monoclonal phages. To facilitate epitope analysis, hIL-1βQ15G mutant that does not bind to IL1RI was constructed, expressed, purified, and identified (Fig. 11A, B, C). Phage ELISA results showed that all 16 phage clones could cross-link with WT hIL-1β, of which, 8 clones bound to hIL-1βQ15CK with significantly reduced affinity (Figure 12), suggesting that the antibody clusters in which these 8 clones reside bind the hIL-1βQ15 epitope. It is noted that these phage clones that bind hIL-1βQ15CK or hIL-1βQ15G with no significant affinity difference may also bind the hIL-1βQ15 epitope, just like that for the E64 epitope described above. However, through a single epitope identification method, it was found that these clones can all bind to the hIL-1βQ15 epitope. If the frequency of each cluster of antibodies is weighted, the proportion of antibodies binding to the hIL-1βQ15 epitope among the 96 analyzed clones was up to 60%.

Next, Q15CK-G8scfv-Fc fusion protein (the antibody cluster antibody sequence in which the clone is located has the highest frequency) was constructed, expressed and purified, and its affinities to hIL-1β and hIL-1βQ15CK were 3.8±0.9nM and 2.4±0.6nM, respectively ( Figure 6C), while the affinity with hIL-1βQ15G decreased about 10 times (Figure 13), consistent with the results of phage ELISA (Figure 12). Taking the above results together, we infer that CK-induced epitope-directed antibody responses are independent of the epitope sequence.

### 6. CK-incorporated IL-1β is expected to be used in the development of subunit vaccines

IL-1β is a pro-inflammatory cytokine that binds with IL-1RI and IL1RII (Afonina et al., 2015). Blockade of IL-1β and IL-1RI signaling pathways can be used to treat a series of autoimmune diseases, such as type II diabetes, rheumatoid arthritis, gout, etc. (Dinarello et al., 2012). Some vaccine based on IL-1β have been used to evaluate their possibility as vaccines, but their efficacy need to be clinically verified (Cavelti-Weder et al., 2016; Spohn et al., 2008; Spohn et al., 2014) . Although the antibody response of traditional subunit vaccines can be enhanced by engineering subunit vaccines or by combination of subunit vaccines with adjuvants, the proportion of neutralizing antibody in the total antibody is very low, and is difficult to be upgraded by currently available method.

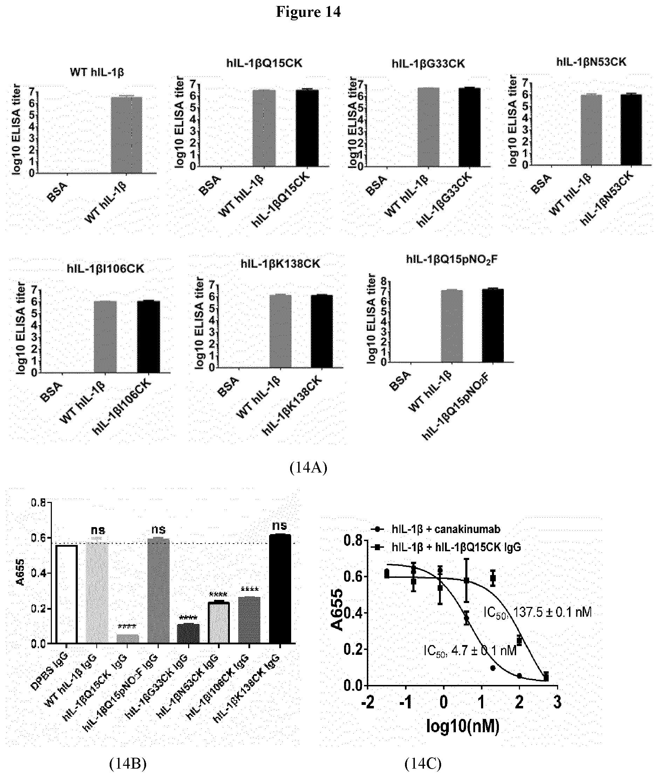

We speculate that if CK is incorporated at the key site of IL-1β binding to IL-1RI, the antibody response elicited by the mutant antigen will target this mutant epitope, and the enriched antibody may blocking the agonistic activity of IL-1β on IL1RI receptor. According to the structure of the hIL-1β-IL1RI (ECD) complex (Vigers et al., 1997), Q15, G33, N53 and I106 are located at the receptor-binding interface, mutants with CK incorporation at these sites(named hIL-1βG33CK, hIL-1βN53CK and hIL-1βI106CK respectively) was constructed, and subjected to mice immunization. IgG antibody titer from WT hIL-1B-immunized and CK-incorporated IL-hIL-1β-immunized mice serum detected 10 days after the third immunization was comparable (~1:10E6 , FIG. 14A). Then, mouse serum were collected, purified by protein A resin, and evaluated for their neutralizing effects. IgG from mice immunized with WT IL-1β or DBPS shown no inhibitory effect, while IgG from mice immunized with hIL-1βQ15CK, hIL-1βG33CK, hIL-1βN53CK or hIL-1βI106CK could significantly inhibit IL-1β-induced activation of HEK-Blue IL-1R (Figure 14B), among which, the IgG from hIL-1βQ15CK-immunized mice had the strongest inhibitory effect, with an IC50 about 137.5±0.1nM. Under the same experimental conditions, the affinity of canakizumab was with IC50 4.7±0.1 nM (FIG. 14C).

hIL-1βK138CK, which was incorporated with CK at non-IL1RI-binding K138 epitope was subjected to mice immunization. Total IgG purified from serum could not inhibit IL-1β from activating HEK-Blue IL-1R. When hIL-1βQ15pNO2F, which was incorporated by pNO2F at Q15 was subjuected to mice immunization, the total IgG in the serum did not show neutralizing activity (Figure 14B), consistent with previous report that TNFa with pNO2F incorporation only increased increased total antibody titers, but not titers of epitope-specific antibodies (Grünewald et al., 2008; Kessel et al., 2014). Our results showed that the incorporation of CK at the interface of IL-1β/IL1RI could induce high-titer neutralizing antibodies which could effectively block the binding of IL-1β and IL1RI, implying great potential for the development of IL-1β vaccine.

### Applicaiton of AK/CK-incorporated novel corona virus RBD in eliciting neutralizing antibodies against specific epitopes and vaccine preparation

### 1. Expression and purification of the RBD region of the novel corona virus S protein and its mutants

Basing on the interaction interface between the novel corona RBD and ACE2 reported in the literature, we selected the K417 site in the mutant strains β and γ, the L452 and Y453 site in the δ and Lambda mutant strain for AK incorporation, while with incorporation at K386 site, a position away from the binding interface with ACE2, as a negative control. Referring to the method of "Expression and Purification of WT IL-1β and its Mutants" in the previous example, the novel corona RBD protein with AK or CK incorporation was expressed in E.coli. After immunized with AK or CK-incorporated RBD protein, B cells produced antibodies against the epitope near AK or CK incorporation elicted by chemical covalent cross-linking, of which, most antibodies were directed against the epitope that binds to ACE2, thereby blocking the binding of RBD with ACE2, and improving the blocking effect of vaccine.

Furthermore, AK or CK-incorporated RBD of novel corona virus S protein was packaged into complete pseudovirus, which can infect 293T-ACE2 cells as observed. In addition, multiple S proteins displayed on the surface of pseudovirus nanoparticles and AK/CK on the S protein can induce production of antibodies against the epitope near the AK or CK incorporation, of which, most are directed against the epitope that binds to ACE2, thereby blocking the binding of RBD with ACE2 and improving the blocking efficacy of the vaccine.

### Peptides incorporated with chemical cross-linking groups enhances the immune response against the peptide

A 20-amino acid polypeptide (sequence: AKPAADNEQSIKPKKKKPKM) (named PTN-WT) of Phaeodactylum tricomutum protein, and a mutant polypeptide (sequence:
APKAADNEQSIK(cr)PKKKKPKM ) (named: PTN-CK) was appended with a Cys at the N-terminus to facilitate coupling in the next step. PTN-WT and PTN-CK were coupled to hemocyanin KLH via N-terminal Cys using SMCC (a bifunctional coupling agent basing on N-hydroxysuccinimide (NHS) active ester and maleimide). Then polypeptides PTN-WT, PTN-CK, KLH-PTN-WT, and KLH-PTN-CK were used to immunize BAlb/C mice, respectively. The adjuvant used for the initial immunization and boost was complete Freund's adjuvant (30ug) and incomplete Freund's adjuvant, respectively. Serum was collected 35 days after immunization, and proteins (such as PTN-WT, PTN-CK, KLH-PTN-WT or KLH-PTN-CK) were individually coated for detecting the titer.

The results are shown in Figure 15: the titer from PTN-CK immunized mice serum was significantly improved compared with that from PTN-WT immunized serum(Figure 15A), suggesting that crotonyl-modified PTN (PTN-CK) can enhance the immunogenicity of the PTN itself; the titers from KLH-PTN-WT or KLH-PTN-CK immunized serum were similar and could reach a higher level(Fig. 15B); the titer against KLH from KLH-PTN-WT immunized serum was significantly higher than that of the KLH-PTN-CK immunized serum(Figure 15C); the antibody against PTN-WT in KLH-PTN-WT immune serum was significantly lower than that in the KLH-PTN-CK immune serum against PTN-CK (Figure 15D); the content of antibodies against PTN-CK in KLH-PTN-WT immune serum was basically similar to that of antibodies against PTN-WT in KLH-PTN-CK immune serum, suggesting that the two groups of serum were cross-reactivity (Fig. 15E).

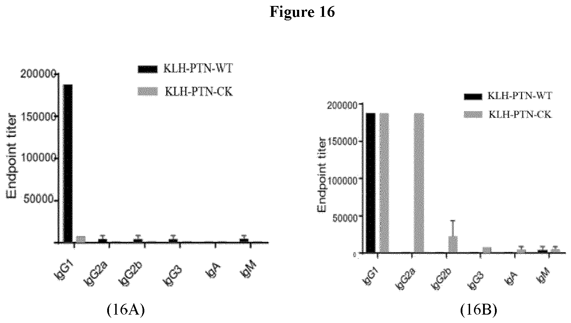

Further, the titers from the immunized sera of the two groups (KLH-PTN-WT, KLH-PTN-CK) against the KLH and PTN polypeptide fractions were compared (Fig. 15F), and results showed that antibodies from KLH-PTN-WT immunized mice are mainly KLH-specific, while antibodies from KLH-PTN-CK-immunized mice were mostly against PTN.
In addition, we further found that the levels of antibodies of different subtypes in the immune sera of the two groups also showed significant differences (Figure 16). Antibodies against KLH protein from KLH-PTN-WT-immunized serum were IgG1 subtype, while antibodies against KLH protein from KLH-PTN-CK were almost undetectable (Figure 16A). The main type of antibody against PTN-WT produced by the KLH-PTN-WT-immunized group was IgG1, while the type of antibody against PTN-CK by the KLH-PTN-CK-immunized group mainly included IgG1, IgG2a, and IgG2b (Figure 16B).

## Claims

1. An agent for immunizing animals comprising:
- an immunologically effective amount of a mutant antigen, which is incorporated with a group with chemical cross-linking activity or a derivative thereof on one or more target epitopes in the wild-type antigen, and
- a physiologically acceptable carrier,
wherein the agent stimulates or enhances the production of antibodies against the one or more target epitopes in vivo after being administered to the animal.

2. The agent according to claim 1, wherein the group with chemical cross-linking activity is a natural amino acid or a non-canonical amino acid.

3. The agent according to claim 1, wherein the group having chemical cross-linking activity is Nε-crotonyl or Nε-acryloyl.

4. The agent according to claim 2, wherein the non-canonical amino acid with a chemical cross-linking active group is Nε-crotonyl-L-lysine (CK) or Nε-acryloyl-L-lysine (AK).

5. The agent according to any one of claims 1-4, wherein the group with chemical cross-linking activity or its derivative is incorporated by genetic codon expansion or chemical synthesis.

6. The agent of any one of claims 1-5, wherein the animal is a rodent, a non-human mammal or a mammal.

7. The agent according to any one of claims 1-6, wherein the wild-type antigen is a soluble protein, a soluble polypeptide, a transmembrane protein expressed on a phospholipid membrane structure, or a polypeptide expressed on a phospholipid membrane structure.

8. The agent according to any one of claims 1 to 7, wherein the agent is an agent for antibody production.

9. The agent according to any one of claims 1-7, wherein the agent is a prophylactic or therapeutic agent.

10. The agent of claim 9, wherein the agent is a vaccine composition.

11. A method for modulating the immunogenicity of an antigen, comprising incorporating groups with chemical cross-linking activity or derivatives thereof on one or more target epitopes of the antigen.

12. The method of claim 11, wherein said modulation results in a change in the profile of the antigen-elicited antibody.

13. A method for improving an animal's immune response against a target epitope of an antigen, wherein the method comprising: administering a certain amount of mutant antigen to the animal, where the mutant antigen is incorporated by chemical cross-linking reactive groups or derivatives on the target epitope of the wild-type antigen.

14. A method for selecting antibodies against a target epitope of a wild-type antigen, the method comprising the steps of
(a) providing a mutant antigen, wherein the mutant antigen is incorporated with a group with chemical cross-linking activity or its derivative thereof on the target epitope of the wild-type antigen;
(b) administering the mutant antigen described in step (a) to the animal;

15. The method of claim 14, further comprising:
(i) isolating serum from said animal;
(ii) using the wild-type antigen to select from the serum for antibodies that specifically bind to the target epitope;
or
(i) isolating B cells from said animal and fusing said B cells with myeloma cells to produce hybridoma cells;
(ii) using the wild-type antigen to select antibodies that specifically bind to the target epitope from the culture supernatant of the hybridoma;
or
(i) isolating B cells from said animal and using them to construct an antibody library;
(ii) using the wild-type antigen to screen from the antibody library for antibodies that specifically bind to the target epitope.

16. The method of claim 14, further comprising:
(i) isolating serum from said animal;
(ii) incubating the mutant antigen with the serum under certain conditions, so that the mutant antigen is covalently cross-linked with the antibody to form a mutant antigen-antibody complex;
or
(i) isolating B cells from said animal and fusing said B cells with myeloma cells to produce hybridoma cells;
(ii) incubating the mutant antigen with the culture supernatant of the hybridoma under certain conditions, so that the mutant antigen is covalently cross-linked with the antibody to form a mutant antigen-antibody complex;
or
(i) isolating B cells from said animal and using them to construct an antibody library;
(ii) incubating the mutant antigen with the antibody library under certain conditions, so that the mutant antigen and antibody are covalently cross-linked to form a mutant antigen-antibody complex;
as well as
(iii) removing antibodies not covalently cross-linked with the mutant antigen by eluting under certain conditions, and releasing antibodies covalently cross-linked with the mutant antigen;
(iv) using the wild-type antigen to further screen antibodies that specifically bind to the target epitope from the antibodies covalently cross-linked with the mutant antigen

17. The method according to claim 16, wherein said incubation conditions are: solution with pH 8.8, temperature: 37° C, time: 24-48 hours.

18. The method of claim 16, wherein said elution conditions are:
(a) alkaline elution with a high pH elution buffer;
(b) acidic elution with a low pH elution buffer.

19. The method of claim 16, wherein the release is carried out by enzymatic digestion which release antigen-cross linked antibodies.

20. The method of any one of claims 11-19, wherein the group having chemical cross-linking activity is a natural amino acid or a non-canonical amino acid.

21. The method according to any one of claims 11-19, wherein the group having chemical crosslinking activity is Nε-crotonyl or Nε-acryloyl.

22. The method of claim 20, wherein the non-canonical amino acid is Nε-crotonyl-L-lysine (CK) or Nε-acryloyl-L-lysine (AK).

23. The method according to any one of claims 11-19, wherein the group with chemical cross-linking activity or its derivative is incorporated by genetic codon expansion or chemical synthesis.

24. The method of any one of claims 11-19, wherein the animal is a rodent, a non-human mammal, or a mammal.

25. The method according to any one of claims 11-19, wherein the antigen is a soluble protein, a soluble polypeptide, a transmembrane protein expressed on a phospholipid membrane, or a polypeptide expressed on a phospholipid membrane.

26. Use of the mutant antigen according to any one of claims 14-19 in the preparation of vaccines for preventing and treating diseases.

27. The antibody obtained by the method according to any one of claims 11-25.

28. Antiserum obtained according to the method of any one of claims 11-25, comprising antibodies against said target epitope.

29. The antibody of claim 28, wherein said antibody specifically binds IL-1b.

30. The antibody of claim 29, wherein said antibody comprises the following VH and VL:
a) VH shown in SEQ ID NO.6 and VL shown in SEQ ID NO.4;
b) VH shown in SEQ ID NO. 10 and VL shown in SEQ ID NO.8;
c) VH shown in SEQ ID NO.14 and VL shown in SEQ ID NO.12;
d) VH shown in SEQ ID NO.18 and VL shown in SEQ ID NO.16;
e) VH shown in SEQ ID NO.22 and VL shown in SEQ ID NO.20;
f) VH shown in SEQ ID NO.26 and VL shown in SEQ ID NO.24;
g) VH shown in SEQ ID NO.30 and VL shown in SEQ ID NO.28.
